# EUROPÄISCHE PATENTANMELDUNG

(11) **EP 1 108 420 A1**
(43) Veröffentlichungstag der Anmeldung: **20.06.2001**
(21) Anmeldenummer: 00126147.8
(22) Anmeldetag: 30.11.2000
(51) Int. Cl.: A61K 7/48

(54) **Kosmetische Zubereitungen**

(30) Priorität: 18.12.1999 DE 19961256
(71) Anmelder: Clariant GmbH, 65929 Frankfurt am Main (DE)
(72) Erfinder: Turowski-Wanke, Angelika, Dr., 65779 Kelkheim (DE); Simsch, Waltraud, 65779 Kelkheim (DE)

(57) **Zusammenfassung**

Kosmetische Zubereitungen enthaltend das Zinksalz von 1-Hydroxy-4-ethyl-6-(2,4,4-trimethylpentyl)-2(1H)-pyridon (Zn-Octopirox).

## Beschreibung

Die vorliegende Erfindung betrifft kosmetische Zubereitungen, enthaltend das Zinksalz von 1-Hydroxy-4-methyl-6-(2,4,4-trimethylpentyl)-2(1 H)-pyridon (Zn-Octopirox®) als antimikrobiellen Wirkstoff.

Die antimikrobielle Wirksamkeit von Pyridin-N-Oxid-Derivaten und Pyridon-Derivaten, beispielsweise 6-[4-(4-Chlor-phenoxy)-phenoxymethyl]-1-hydroxy-4-methyl-2-pyridon, 2-Aminoethanolsalz, ist bekannt. In WO 98 55093 werden milde Reinigungsmittel beansprucht, enthaltend antimikrobiell wirkende Additive wie z.B Zinkpyrithion und Octopirox (Pirocton Olamin).

Es wurde nun gefunden, daß das Zinksalz von 1-Hydroxy-4-methyl-6-(2,4,4-trimethylpentyl)-2(1 H)-pyridon (Zink-Octopirox ) in kosmetischen Mitteln ein breites Wirkungsspektrum gegenüber Mikroorganismen zeigt, die sowohl die Haut als auch die Schleimhaut befallen, wie Bakterien, Hefen sowie Pilze. In Gegenwart des erfindungsgemäß eingesetzten Zink-Octopirox kann auf zytotoxische Agentien verzichtet werden.
Die erfindungsgemäßen Zubereitungen enthalten den antimikrobiell wirkenden Wirkstoff Zink-Octopirox in den Gewichtsmengen von 0,05 % bis 10 %, bevorzugt von 0,05 % bis 5 %, besonders bevorzugt von 0,1 % bis 1,0 %.

Das in den kosmetischen Zubereitungen einzusetzende Zinksalz von 1-Hydroxy-4-methyl-6-(2,4,4-trimethylpentyl)-2(1H)-pyridon kann nach gängigen Verfahren durch Umsetzung von 1-Hydroxy-4-methyl-6-(2,4,4-trimethylpentyl)-2(1H)-pyridon bzw. dessen Salz, insbesondere dessen -Aminoethanolsalz und einem Zinksalz, beispielsweise ZnCl₂, hergestellt werden. Das Zinksalz von 1-Hydroxy-4-methyl-6-(2,4,4-trimethylpentyl)-2(1H)-pyridon kann auch "in situ" hergestellt werden durch Zugabe eines anorganischen Zinksalzes, insbesondere eines wasserlöslichen Zinksalzes, besonders bevorzugt ZnCl₂, zu einer Formulierung, enthaltend das Salz des 1-Hydroxy-4-methyl-6-(2,4,4-trimethylpentyl)-2(1H)-pyridons, beispielsweise das Na-, K-, Mg-, Ca-Salz, insbesondere das 2-Aminoethanolsalz des 1-Hydroxy-4-methyl-6-(2,4,4-trimethylpentyl)-2(1H)-pyridons (Octopirox) oder die entsprechende freie Säureform dieser Verbindung. Hierbei werden Octopirox und Zn-Salz, insbesondere ZnCl₂ in molaren Verhältnissen von 1 zu 20 bis 20 zu 1, bevorzugt von 5 zu 1 bis 1 zu 5, besonders bevorzugt 1 zu 2,5 eingesetzt. Die Umsetzung von 1-Hydroxy-4-methyl-6-(2,4,4-trimethylpentyl)-2(1H)-pyridon-Salzen, insbesondere des 2-Aminoethanolsalzes und ZnCl₂ kann unvollständig sein, so dass in den kosmetischen Zubereitungen eine Mischung aus nicht umgesetztem 1-Hydroxy-4-methyl-6-(2,4,4-trimethylpentyl)-2(1H)-pyridon-Salz, insbesondere 2-Aminoethanolsalz, dem Zinksalz und ZnCl₂ vorliegt.
1-Hydroxy-4-methyl-6-(2,4,4-trimethylpentyl)-2(1H)-pyridon bzw. dessen Salze aus anorganischen und organischen Säuren werden nach den in EP 241 918,
EP 646 368, DE 17 95 270, DE 22 14 608 und DE 22 34 009 beschriebenen Methoden erhalten.

Für den erfindungsgemäßen Einsatz des Zn-Octopirox kommen die unterschiedlichsten kosmetischen Zubereitungen in Betracht, insbesondere Shampoos, Kopfhautcremes, desodorierende Körperreinigungsmittel, Pflegemittel und Emulsionen. Als Beispiele für weitere erfindungsgemäß in Betracht kommende Zubereitungen seien folgende erwähnt: Haarspülmittel, Haarkuren, Haarregeneriermittel, Haarwässer, Wasserwellotionen, Haarsprays, Frisiercremes, Frisiergels, Haaröle, Haarpomaden, Haarbrillantinen, Duschbäder, Seifen, Flüssigseifen, Deo-Sticks, Deo-Spray, Emulsionen. Es handelt sich demnach immer um Zubereitungen, die je nach ihrem eigentlichen Anwendungszweck für kürzere oder längere Zeit auf das Haar, auf die Kopfhaut oder auf die Haut aufgebracht werden.
Desweiteren können die erfindungsgemäßen Zubereitungen "leave on" Produkte, beispielsweise konditionierende Haarcremes, Fußsprays- oder -cremes, antibakterielle Gesichtswässer oder Körperlotions sein.
Werden die erfindungsgemäßen kosmetischen Zubereitungen als Shampoos, Duschbäder oder Lotionen dargeboten, so können diese klarflüssig, opakflüssig (mit
Perlglanzeffekt), cremeförmig, gelartig, pulverförmig oder in Tablettenform, sowie als Aerosole konditioniert sein.
Die diesen Mitteln zugrunde liegenden Waschrohstoffe können anionischer, kationischer, nichtionischer und amphoterer Natur sein und auch in Kombination miteinander vorliegen. Die Gesamtmenge der in den erfindungsgemäßen Mitteln eingesetzten Tensiden kann zwischen 5 und 70 Gew.-%, bevorzugt zwischen 10 und 40 Gew.-%, besonders bevorzugt zwischen 12 und 35 Gew.-%, bezogen auf das fertige Mittel, sein.

Als Beispiele für derartige anionische waschaktive Substanzen seien genannt:
C₁₀-C₂₀-Alkyl- und Alkylen-carboxylate, Alkylethercarboxylate, Fettalkoholsulfate,
Fettalkoholethersulfate, Alkylolamidsulfate und -sulfonate, Fettsäurealkylolamidpolyglykolethersulfate, Alkansulfonate und Hydroxyalkansulfonate, Olefinsulfonate, Acylester von Isethionaten, a-Sulfofettsäureester, Alkylbenzolsulfonate,
Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, sowie Acylglutamate. Diese Verbindungen und deren Mischungen werden in Form ihrer wasserlöslichen oder wasserdispergierbaren Salze benutzt, beispielsweise in Form der Natrium-, Kalium-, Magnesium-, Ammonium-, Mono-, Di- und Triethanolammonium- sowie analogen Alkylammonium-Salze.
Der Gewichtsanteil der anionischen Tenside in den erfindungsgemäßen Mitteln liegt im Bereich von 7 bis 30 %, bevorzugt 10 bis 25 %, besonders bevorzugt 12 bis 22 %.

Geeignete kationische Tenside sind beispielsweise quartäre Ammonium-Salze wie Di-(C₁₀-C₂₄-Alkyl)-dimethyl-ammonium-chlorid oder -bromid, vorzugsweise Di-(C₁₂-C₁₈-Alkyl)-dimethyl-ammonium-chlorid oder -bromid; C₁₀-C₂₄-Alkyl-dimethylethylammonium-chlorid oder -bromid; C₁₀-C₂₄-Alkyl-trimethyl-ammonium-chlorid oder -bromid, vorzugsweise Cetyl-trimethyl-ammonium-chlorid oder -bromid und C₂₀-C₂₂-Alkyl-trimethyl-ammonium-chlorid oder -bromid; C₁₀-C₂₄-Alkyl-dimethylbenzylammonium-chlorid oder -bromid, vorzugsweise C₁₂-C₁₈-Alkyl-dimethylbenzylammonium-chlorid; N-(C₁₀-C₁₈-Alkyl)-pyridinium-chlorid oder -bromid, vorzugsweise N-(C₁₂-C₁₆-Alkyl)-pyridinium-chlorid oder -bromid; N-(C₁₀-C₁₈-Alkyl)-isochinoliniumchlorid, -bromid oder -monoalkylsulfat; N-(C₁₂-C₁₈-Alkyloylcolaminoformylmethyl)-pyridinium-chlorid; N-(C₁₂-C₁₈-Alkyl)-N-methyl-morpholinium-chlorid, -bromid oder -monoalkylsulfat; N-(Cᵢ₂-C₁₈-Alkyl)-N-ethyl-morpholinium-chlorid, -bromid oder -monoalkylsulfat; C₁₆-C₁₈-Alkyl-pentaoxethyl-ammonium-chlorid; Diisobutylphenoxyethoxyethyldimethylbenzylammonium-chlorid; Salze des N,N-Diethylaminoethylstearylamids und -oleylamids mit Salzsäure, Essigsäure, Milchsäure, Zitronensäure, Phosphorsäure; N-Acyl-amidoethyl-N,N-diethyl-N-methylammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.
Der Gewichtsanteil der kationischen Tenside in den erfindungsgemäßen Mitteln liegt im Bereich von 1 bis 10 %, bevorzugt 2 bis 7 %, besonders bevorzugt 3 bis 5 %.
Als nichtionische Tenside, die als waschaktive Substanzen eingesetzt werden können, kommen beispielsweise in Betracht: Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole);
Polypropylenglykolethoxylate (Pluronic); Fettsäurealkylolamide (Fettsäureamidpolyethylenglykole); N-Alkyl-, N-Alkoxypolyhydroxyfettsäureamid, Saccharoseester; Sorbitester und Polyglykolether.
Der Gewichtsanteil der nichtionischen Tenside in den erfindungsgemäßen Mitteln liegt im Bereich von 1 bis 20 %, bevorzugt 2 bis 10 %, besonders bevorzugt 3 bis 7 %.

In den erfindungsgemäßen Mitteln einsetzbar sind Amphotenside, beispielsweise:
N-(C₁₂-C₁₈-Alkyl)-β-aminopropionate und N-(C₁₂-C₁₈-Alkyl)-β-iminodipropionate als Alkali- und Mono-, Di- und Trialkylammonium-Salze; N-Acylamidoalkyl-N,N-dimethylacetobetain, vorzugsweise N-(C8-C₁₈-Acyl)amidopropyl-N,N-dimethylacetobetain;
C₁₂-C₁₈-Alkyl-dimethyl-sulfopropyl-betain; Amphotenside auf Basis Imidazolin (Handelsname: Miranol®, Steinapon®), vorzugsweise das Natrium-Salz des 1-(β-Carboxy-methyloxyethyl)-1-(carboxymethyl)-2-lauryl-imidazoliniums; Aminoxid, z.B. C₁₂-C₁₈-Alkyldimethylaminoxid, Fettsäureamidoalkyl-dimethylaminoxid.

Der Gewichtsanteil der amphoteren Tenside in den erfindungsgemäßen Mitteln liegt im Bereich von 0,5 bis 20 %, bevorzugt 1 bis 10 %.

Desweiteren können in den erfindungsgemäßen Mitteln schaumverstärkende Co-Tenside aus der Gruppe Alkylbetaine, Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Bevorzugte Tenside in den erfindungsgemäßen Mitteln sind Laurylsulfat, Laurethsulfat, Cocoamidopropylbetain, Natriumcocoylglutamat,
Di-natriumlaurethsulfosuccinat und Cocosfettsäurediethanolamid.

Die erfindungsgemäßen Zubereitungen können außerdem weitere in der Kosmetik gebräuchliche Zusätze enthalten wie z.B. Emulgatoren, Co-Emulgatoren, Überfettungsmittel, Fette, Öle, Wachse, Stabilisatoren, biogene Wirkstoffe, Glycerin, Konservierungsmittel, Perlglanzmittel, Farb- und Duftstoffe, Lösungsmittel, Trübungsmittel, Verdickungsmittel und Dispergiermittel, Eiweißderivate, wie Gelatine, Collagenhydrolysate, Polypeptide auf natürlicher und synthetischer Basis, Eigelb, Lecithin, Lanolin und Lanolinderivate, Fettalkohole, Silicone, Silicon-Derivate, beispielsweise polyquaternierte Polysiloxan-Polymere, insbesondere Polyquaternium-4, -6, -7, -10, -15, Siliconöl, Dimethicone, beispielsweise Diphenyldimethicone, Phenyltrimethicone, Polymere mit dreidimensionalen Netzstrukturen, desodorierende Mittel, Stoffe mit keratolytischer und keratoplastischer Wirkung, Enzyme und Trägersubstanzen. Desweiteren können den erfindungsgemäßen Mitteln weitere antimikrobiell wirkende Agentien anderer Struktur zugesetzt werden.
Als Emulgatoren können beispielsweise eingesetzt werden: Sorbitanester, Sorbitolester, Phosphorsäureester, Monoglyceride, Polysorbate, Polyethylenglycolmono/difettsäureester, hochethoxylierte Fettsäureester sowie hochmolekulare Siliconverbindungen, wie z.B. Dimethylpolysiloxane mit einem durchschnittlichen Molekulargewicht von 10.000 bis 50.000.

Als nichtionogene O/W-Co-Emulgatoren kommen in Betracht Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; C₁₂-C₁₈-Fettsäuremono- und -diester von Anlagerungsprodukten von 1 bis 30 Mol Ethylenoxid an Glycerin, Glycerinmono- und -diester und Sorbitanmono- und -diester von gesättigten und ungesättigten Fettsäuren mit 6 bis 22 Kohlenstoffatomen und deren Ethylenoxidanlagerungsprodukten; Anlagerungsprodukte von 15 bis 60 Mol Ethylenoxid an Rizinusöl und/oder gehärtetes Rizinusöl, Polyol- und insbesondere Polyglycerinester, wie z.B. Polyglycerinpolyricinoleat und Polyglycerinpoly-12-hydroxystearat. Ebenfalls geeignet sind Gemische von Verbindungen aus mehreren dieser Substanzklassen. Die Anlagerungsprodukte von Ethylenoxid und/oder Propylenoxid an Fettalkohole, Fettsäuren, Alkylphenole, Glycerin-mono- und -diester sowie Sorbitanmono- und -diester oder Sorbitolmono- und -diester von Fettsäuren oder an Rizinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C₁₂-C₁₈-Fettsäuremono- und -diester von Anlagerungsprodukten von Ethylenoxid an Glycerin sind aus DE 20 24 051 als Rückfettungsmittel für kosmetische Zubereitungen bekannt.

Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für Fette sind Glyceride, als Wachse kommen u.a. Bienenwachs, Paraffinwachs oder Mikrowachse, gegebenenfalls in Kombination mit hydrophilen Wachsen, z.B. Cetylstearylalkohol in Frage.

Als Stabilisatoren können Metallsalze von Fettsäuren, wie z.B. Magnesium- und/oder Zinkstearat eingesetzt werden.
Unter biogenen Wirkstoffen sind beispielsweise Pflanzenextrakte und Vitaminkomplexe zu verstehen.
Als Konservierungsmittel eignen sich beispielsweise Phenoxyethanol, Formaldehydlösung, Parabene, Pentandiol oder Sorbinsäure. Als Perlglanzmittel kommen beispielsweise Glycoldistearinsäureester, wie Ethylenglycoldistearat, aber auch Fettsäuremonoglycolester in Betracht.
Als Verdickungsmittel eignen sich Natrium-, Kalium-, Ammoniumchlorid, Natriumsulfat, Fettsäurealkylolamide, Cellulosederivate, beispielsweise Hydroxyethylcellulose, Guar Gum, Polyvinylalkohol, Polyvinylpyrrolidon, Hydroxypropyl guar gum, Stärke und Stärkederivate sowie natürliche Gummen, Carboxyvinylpolymere, beispielsweise Carbopol 934, -940, -941,-956,-980,-981, -1342, -1382.
Als Verdickungs- und Dispergiermittel werden eingesetzt Acylderivate und Aminoxide mit langkettigen, aliphatischen Gruppen mit 8-22, bevorzugt 14-22, besonders bevorzugt 16-22 Kohlenstoffatomen, und Mischungen daraus, ebenso langkettige Ethylenglycolester, Alkanolamide, langkettige Fettsäureester, Glycerinester, Alkanolamidester und Alkyldimethylaminoxide, sowie die in US 4,741,855 benannten Dispergiermittel.
Für Schampoos besonders geeignet sind Ethylenglycolester von Fettsäuren mit 14 bis 22, besonders bevorzugt 16 bis 22 Kohlenstoffatomen, insbesondere Mono- und Di-ethylenglycolstearat. Bovorzugt sind auch Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat, N,N-Dihydrocarbyl (C₁₂-C₂₂), insbesondere C₁₆-C₁₈)-amidobenzoesäure und deren lösliche Salze, N,N-di-(C₁₆-C₁₈)amidobenzoe-säure und Derivate.
Die Dispergiermittel werden in Konzentrationen von 0,5 bis 10 Gew.-%, bevorzugt von 0,5 bis 5 Gew.-%, besonders bevorzugt von 1 bis 4 Gew.-% bezogen auf das fertige Mittel eingesetzt.
Als Trägermaterialien in Betracht kommen pflanzliche Öle,natürliche und gehärtete Öle, Wachse, Fette, Wasser, Alkohole, Polyole, Glycerol, Glyceride, flüssige Paraffine, flüssige Fettalkohole, Sterol, Polyethylenglykole, Cellulose und Cellulose-Derivate.

Als antifungizide Wirkstoffe können Ketoconazol, Oxiconazol, Terbinafin, Bifonazole,Butoconazole, Cloconazole, Clotrimazole, Econazole, Enilconazole, Fenticonazole, Isoconazole, Miconazole, Sulconazole, Tioconazole, Fluconazole, Itraconazole, Terconazole und Naftifine eingesetzt werden.

Um die Affinität des erfindungsgemäß eingesetzten Zinksalzes von 1-Hydroxy-4-methyl-6-(2,4,4-trimethylpentyl)-2(1H)-pyridon auf der Haut/Kopfhaut zu verbessern können Polymere, beispielsweise kationische Guar Polymere in den Gewichtsmengen 0,01 bis 1,0 %, bevorzugt 0,02 bis 0,25 % eingesetzt werden, wie in WO 97/26854 beschrieben.

Die antimokrobielle Wirkung des erfindungsgemäß eingesetzten Zinksalzes von 1-Hydroxy-4-methyl-6-(2,4,4-trimethylpentyl)-2(1H)-pyridon kann durch Zugabe von Polyethylenglycol (PEG), insbesondere mit 6 bis 22 EO-Gruppen, bevorzugt PEG-12, Polypropylenglycol (PPG), Polyethoxy/polypropoxy Copolymere, Polyethylenimine, polyethoxylierte Polyethylenimine, Polyethylenoxidfettsäureglyceride, ethoxyliertes Nonylphenol, ethoxylierte Alkohole, Polyethylencarbohydrate verbessert werden.
Silicone, Polyalkylsiloxane, Polyalkylarylsiloxane, Polyethersiloxan-Copolymere, wie in US 5104645 und den darin zitierten Schriften beschrieben, verbessern die pflegende Wirkung der erfindungsgemäßen Mittel.

Die Herstellung der erfindungsgemäßen Mittel erfolgt in an sich bekannter Weise durch Zusammengeben der einzelnen Komponenten und einer - soweit erforderlich - der jeweiligen Zubereitungsart angepaßten Weiterverarbeitung. Einige dieser vielfältigen möglichen Zubereitungsformen werden in den Ausführungsbeispielen beispielhaft beschrieben.

Auch die Herstellung aller dieser Zubereitungen erfolgt - wie bereits beim Shampoo erwähnt - in an sich bekannter Weise unter Zugabe des erfindungsgemäß eingesetzten Wirkstoffs.
In die erfindungsgemäßen Zubereitungen wird der Antischuppenwirkstoff in Mengen eingearbeitet, die üblicherweise zwischen etwa 0,05 und etwa 10 % liegen. Innerhalb dieses Bereiches richten sich die Konzentrationen der speziellen Zubereitungen nach ihrem Anwendungszweck. Bestimmte Zubereitungsformen, wie z.B. Konzentrate, die vor ihrer Anwendung zu verdünnen sind, können auch erheblich höhere Konzentrationen aufweisen.

Handelt es sich um Zubereitungen, die auf dem Haar verbleiben, wie beispielsweise Haarwasser, Haarfestiger, Cremes usw., so wird man niedrigere Konzentrationen einsetzen, beispielsweise von ca. 0,01 bis ca. 1%, vorzugsweise 0,1 bis 0,5 Gew.-%. In höheren Konzentrationen werden sie zweckmäßigerweise dann zur Anwendung kommen, wenn es sich um kosmetische Zubereitungen handelt, die, gegebenenfalls nach Verdünnung, nur kurze Zeit auf Haar und Kopfhaut einwirken, wie beispielsweise Shampoos oder Haarspülmittel. In diesen Fällen können z.B. Konzentrationen von etwa 0,2 bis etwa 10 %, vorzugsweise etwa 0,5 bis etwa 2 Gew.-%, zweckmäßig sein.

Die erfindungsgemäße Verwendung des genannten Zinksalzes von 1-Hydroxy-4-methyl-6-(2,4,4-trimethylpentyl)-2(1H)-pyridon zeichnet sich insbesondere gegenüber dem erwähnten Stand der Technik durch zahlreiche Vorteile aus.

Herauszuheben sind:
- verbesserte Substantivität, dadurch geringe Wirkstoffkonzentrationen
- breites antimikrobielles Wirkungsspektrum
- gute Löslichkeit in wässrigen und nicht-wässrigen Medien

Die nachstehenden Beispiele erläutern die vorliegende Erfindung. Die Mengenangaben sind auf das Gewicht bezogen.

### Beispiel 1: Antischuppenschampoo

| Komponente | | Gew.-% |
|---|---|---|
| A | Octopirox | 0,50 |
| B | Wasser | 10,00 |
| C | Genapol LRO flüssig | 30,00 |
| D | Belsil DMC 6032 | 0,50 |
| E | Allantoin | 0,30 |
| F | Wasser | ad 100 |
| G | Panthenol | 1,00 |
| | Genagen CAB | 8,00 |
| | Genapol L-3 | 1,50 |
| | Farbstoff | q.s. |
| H | NaCI | 1,50 |
| I | ZnCl₂ | 0,35 |

### Herstellweise:

I A in B einrühren
II C in I einrühren und bis zum Klarwerden der Lösung weiterrühren
III D portionsweise dazugeben
IV E in F lösen und unter Rühren und leichter Erwärmung zugeben
V Komponenten von G nacheinander unterrühren
VI falls erforderlich, den pH einstellen
VII Viskosität mit H einstellen
VIII I unterrühren

### Beispiel 2: Antischuppenschampoo

| Komponente | | Gew.-% |
|---|---|---|
| A | Octopirox | 0,50 |
| B | Wasser | 10,00 |
| C | Genapol LRO flüssig | 30,00 |
| D | Belsil DMC 6032 | 0,50 |
| E | Allantoin | 0,30 |
| | Polyquaternium 10 | 0,20 |
| F | Wasser | ad 100 |
| G | Panthenol | 1,00 |
| | Genagen CAB | 8,00 |
| | Genapol L-3 | 1,50 |
| | Farbstoff | q.s. |
| H | NaCI | 1,50 |
| I | ZnCl₂ | 0,35 |

### Herstellweise:

I A in B einrühren
II C in I einrühren und bis zum Klarwerden der Lösung weiterrühren
III D portionsweise dazugeben
IV E in F lösen und unter Rühren und leichter Erwärmung zugeben V Komponenten von G nacheinander unterrühren
VI falls erforderlich, den pH einstellen
VII Viskosität mit H einstellen
VIII I unterrühren

### Beispiel 3: Antischuppenschampoo

| Komponente | | Gew.-% |
|---|---|---|
| A | Octopirox | 0,50 |
| B | Wasser | 10,00 |
| C | Genapol LRO flüssig | 30,00 |
| D | Belsil DMC 6032 | 0,50 |
| E | Allantoin | 0,30 |
| F | Wasser | ad 100 |
| | Merquat 550 | 0,50 |
| G | Panthenol | 1,00 |
| | Genagen CAB | 8,00 |
| | Genapol L-3 | 1,50 |
| | Farbstoff | q.s. |
| H | NaCI | 1,50 |
| I | ZnCl₂ | 0,35 |

### Herstellweise:

I A in B einrühren
II C in I einrühren und bis zum Klarwerden der Lösung weiterrühren
III D portionsweise dazugeben
IV E in F lösen und unter Rühren und leichter Erwärmung zugeben
V Komponenten von G nacheinander unterrühren
VI falls erforderlich, den pH einstellen
VII Viskosität mit H einstellen
VIII I unterrühren

### Beispiel 4: Antischuppenschampoo, Intensiv-Schampoo

| 15 Komponente | | Gew.-% |
|---|---|---|
| A | Octopirox | 0,75 |
| B | Wasser | 10,00 |
| C | Genapol LRO flüssig | 35,00 |
| | Genapol SBE | 10,00 |
| D | Parfüm | 0,30 |
| | Wasser | ad 100 |
| | Genagen CAB | 6,00 |
| | Farbstoff | q.s. |
| E | NaCI | 1,70 |
| F | ZnCl₂ | 0,525 |

### Herstellweise:

I A in B einrühren
II C in I einrühren und bis zum Klarwerden der Lösung weiterrühren
30 III D portionsweise dazugeben
IV falls erforderlich, den pH einstellen
V Viskosität mit E einstellen
VII unterrühren

### Beispiel 5: Antischuppenschampoo mit Perlglanzeffekt

| Komponente | | Gew.-% |
|---|---|---|
| A | Genapol 26 L 80 | 0,50 |
| B | Dow Corning 190 | 0,20 |
| C | Octopirox | 0,40 |
| D | Wasser | 5,00 |
| E | Genapol LRO flüssig | 35,00 |
| F | Hostapon CCG | 2,00 |
| | Parfüm | 0,30 |
| | Glycerin | 1,00 |
| | Genapol TSM | 3,00 |
| | Genagen LAA | 10,00 |
| | Merquat 550 | 0,50 |
| G | Cosmedia Guar | 0,10 |
| H | Wasser | ad 100 |
| | Zitronensäure | 0,30 |
| I | DOE 120 | 1,00 |
| | Konservierung | q.s. |
| J | NaCI | 0,50 |
| K | ZnCl₂ | 0,28 |

### Herstellweise:

I B in A unter leichtem Erwärmen lösen
II C mit D verrühren, E zugeben und rühren bis eine klare Lösung vorliegt
III nacheinander die Komponenten von F in II einrühren
IV I in III einrühren
V G in H mit Ultra Turrax lösen und in IV einrühren
VI I in V einrühren und solange rühren bis alles gelöst ist
VII falls erforderlich, den pH einstellen (5,5 - 6,0)
VIII mit J die Viskosität einstellen
IX k unterrühren

### Beispiel 6: Instant Vitamin Antischuppen Haarkur

| Komponente | | Gew.-% |
|---|---|---|
| A | Propylenglykol | 0,70 |
| B | Octopirox | 0,05 |
| C | Carbopol 980 | 0,60 |
| D | Genamin KDM-P | 0,20 |
| | Genamin CTAC | 0,50 |
| | Weizenstärke | 0,50 |
| | Belsil DMC 6032 | 0,80 |
| | Dow Corning 190 | 0,50 |
| E | Wasser | ad 100 |
| | Panthenol | 0,20 |
| | Celquat L 200 | 0,10 |
| | Uvinol MS 40 | 0,05 |
| | Nicotinamide | 0,30 |
| | Vitamin E | 0,10 |
| | Natronlauge | 0,80 |
| F | Cosmedia Guar | 0,20 |
| G | Parfüm | 0,30 |
| H | ZnCl₂ | 0,035 |

### Herstellweise:

I B in A lösen
II D bei ca. 80°C aufschmelzen
III F in E mit Ultra Turrax einrühren
IV III auf 80°C erwärmen
V C zu II geben und direkt anschließend IV einrühren
VI kaltrühren
VII bei ca. 35°C einrühren

### Beispiel 7: Cremeshampoo

| Komponente | Gew.-% |
|---|---|
| Zn-Octopirox | 0,20 |
| Hostapon CT-Paste | 70 |
| Hostapon SCI-65 | 5 |
| Medialan LDD | 5 |
| Ethylenglycoldistearat | 1,50 |
| Wasser | ad 100 |

### Herstellweise:

### Beispiel 8: Antischuppenschampoo

| Komponente | Gew.-% |
|---|---|
| Zn-Octopirox | 0,30 |
| Genagen CA 050 | 5 |
| Genapol LRO, fl | 35,0 |
| Genapol AMG | 10,0 |
| Genagen CAB | 8,0 |
| Genapol L-3 | 2,0 |
| Natriumchlorid | 0,7 |
| Wasser | ad 100 |

### Herstellweise:

### Beispiel 9: 3 in 1-Shampoo

| Komponente | Gew.-% |
|---|---|
| Zn-Octopirox | 0,20 |
| Genapol LRO, fl | 35,0 |
| Genapol AMG | 5,0 |
| Hostapon KCG | 5,0 |
| Hostapon SCHC | 5,0 |
| Belsin DNC 6032 | 2,0 |
| D-Panthenol | 1,0 |
| Genapol L-3 | 2,5 |
| Genagen CAB | 8,0 |
| Natriumchlorid | 2,0 |
| Wasser | ad 100 |

Chemische Bezeichnung der eingesetzten Handelsprodukte

| | | |
|---|---|---|
| ®Octopirox | Clariant GmbH | 1-Hydroxy-4-methyl-6-(2,4,4 trimethylpentyl)-2(1 H)-pyridon, 2-Aminoethanolsalz |
| | | |
| | | |
| ®Genapol LRO | Clariant GmbH | Natriumlaurethsulfat |
| ®Belsin DNC 6032 | Wacker Chemie | Dimethicon-Copolyol-Acetat |
| Allantoin | Clariant GmbH | 5-Ureido-hydantoin |
| ®Genagen CAB | Clariant GmbH | Cocoamidopropylbetain |
| Genapol L-3 | Clariant GmbH | Fettalkoholpolyglycolether, 3 EO |
| ®Merquart 550 | | Polyquaternium 7 |
| Genapol SBE | Clariant GmbH | Alkylpolyglykolethersulfosuccinat |
| Genapol 26 L 80 | Clariant GmbH | Fettalkoholpolyglycolether, 8 EO |
| Dow Corning 190 | | Dimethicon Copolyol |
| Hostapon KCG | Clariant GmbH | Natriumcocoylglutamat |
| Genapol TSM | Clariant GmbH | PEG-3 Distearat/Natriumlaurethsulfat |
| Genagen LAA | Clariant GmbH | Natriumlauroamphoacetat Guar Hydroxypropyltrimethyl ammoniumchlorid |
| ®Cosmedia Guar | | |
| | | |
| ®Gulcamat DOE 120 | | PEG-120 Methylglucosedioleat |
| ®Carbopol 980 | | Carbomer |
| Genamin KDM-P | Clariant GmbH | Behenyltrimethylammoniumchlorid |
| Genamin CTAC | Clariant GmbH | Cetyltrimethylammoniumchlorid |
| ®Celquat L 200 | | Polyquaternium-4 |
| ®Uvimol MS 40 | | Benzophenon-4 |
| Vitamin E | | Tocopherolacetat |
| Hostapon CT-Paste | Clariant GmbH | Fettsäuremethyltaurid-Natriumsalz |
| Hostapon SCI-65 | Clariant GmbH | Fettsäuremethylisethionat Natriumsalz |
| | | |
| ®Medialan LD | Clariant GmbH | Palmkernfettsäuresarkosid |
| Genagen CA 050 | Clariant GmbH | Fettsäuremonoethanolamid polyglykolether |
| | | |
| Genapol AMG | Clariant GmbH | Acylaminopolyglykolethersulfat Magnesiumsalz |
| | | |
| Hostapon SCHC | Clariant GmbH | Fettsäure-Protein-Kondensat |
| Belsin DNC 6032 | Wacker Chemie | Silikonöl |

## Patentansprüche

1. Kosmetische Zubereitungen, dadurch gekennzeichnet,dass sie das Zinksalz von 1-Hydroxy-4-ethyl-6-(2,4,4-trimethylpentyl)-2(1 H)-pyridon enthalten.

2. Kosmetische Zubereitungen nach Anspruch 1, dadurch gekennzeichnet, dass sie zusätzlich das 2-Aminoethanolsalz von 1-Hydroxy-4-methyl-6-(2,4,4-trimethylpentyl)-2(1H)-pyridon sowie ein anorganisches Zinksalz enthalten.

3. Kosmetische Zubereitungen nach Anspruch 1, dadurch gekennzeichnet, dass sie zusätzlich das Na- oder K- oder Mg- oder Ca-Salz von 1-Hydroxy-4-methyl-6-(2,4,4-trimethylpentyl)-2(1H)-pyridon sowie ein anorganisches Zinksalz enthalten.

4. Kosmetische Zubereitungen nach Anspruch 1, dadurch gekennzeichnet, dass sie 0,05 bis 10 Gew.-% des Zinksalzes von 1-Hydroxy-4-methyl-6-(2,4,4-trimethylpentyl)-2(1 H)-pyridon enthalten.
